Europäisches Patentamt

European Patent Office

Office européen des brevets

(11) Numéro de publication: **0 395 555**
**A1**

## (12) DEMANDE DE BREVET EUROPEEN

(21) Numéro de dépôt: 90460013.7

(22) Date de dépôt: 07.03.90

(51) Int. Cl.⁵: **G06F 15/20**

(30) Priorité: 07.03.89 FR 8903131

(43) Date de publication de la demande:
**31.10.90 Bulletin 90/44**

(84) Etats contractants désignés:
**DE FR GB NL**

(71) Demandeur: **EERL SARL**
**80 Avenue des Buttes de Coesmes**
**F-35700 Rennes(FR)**

(72) Inventeur: **Gosselin, Annie**
**16 rue de la Rivière**
**F-35760 Saint-Gregoire(FR)**

Inventeur: **Le Pichon, Jean-Pierre**
**16 Allée de la Maille**
**F-35690 Acigne(FR)**
Inventeur: **Quero, Jean-Claude**
**73 Boulevard de Sévigné**
**F-35700 Rennes(FR)**
Inventeur: **Geille, Christian**
**1 Rue du Grand Champ**
**F-35510 Cesson Sevigne(FR)**

(74) Mandataire: **Corlau, Vincent**
**c/o Cabinet Vidon Immeuble Germanium 80**
**avenue des Buttes de Coesmes**
**F-35700 Rennes(FR)**

(54) **Procédé d'analyse d'un signal, notamment en manométrie digestive.**

(57) L'invention concerne un système d'acquisition automatique de données de manométrie digestive, et un système informatisé d'interprétation de ces données. Le système a pour objectif d'être susceptible d'une mise en oeuvre dans un matériel commercialisable, compatible, conversationnel et configurable.

L'objectif est de fournir un système automatisé, visant à permettre la réduction du temps de dépouillement des résultats, et l'obtention de fonctions performantes de représentation graphique, d'analyse et d'archivage des données.

Cet objectif est atteint à l'aide d'un procédé comprenant les étapes de correction de la ligne de base de la courbe représentative du signal étudié, (en tenant compte des périodes de tonus musculaire), de filtrage, d'élimination des artefacts, de détection des zones d'activité et de linéarisation de ladite courbe.

Fig. 2

## Procédé d'analyse d'un signal, notamment en manométrie digestive.

L'invention concerne un système d'acquisition automatique de données de manométrie digestive, et un système informatisé d'interprétation de ces données. Le système a pour objectif d'être susceptible d'une mise en oeuvre dans un matériel commercialisable, compatible, conversationnel et configurable.

On peut distinguer deux familles principales d'analyse en motricité digestive, sous deux types de modalités (statique ou ambulatoire) :

. L'analyse de la motricité oesophagienne et ano-rectale, réalisée par acquisition dynamique (déplacement d'une sonde sur 15 à 60 cm environ) au cours d'un examen statique de 20 minutes à une heure environ ;

. L'analyse de la motricité colique et de la motricité de l'intestin grêle (duodénale, jéjunale et iléale), au cours d'un examen statique de 3 à 24 heures environ en relevés continus et sonde fixe.

Les versions ambulatoires de ces analyses de motricité s'effectuent par cycles de 24 heures.

Les sondes utilisées sont constituées de 3 à 8 voies d'acquisition, à partir de capteurs de pressions, in situ (jauges de contraintes) ou externes et connectés à des cathéters perfusés à orifices étagés. Les signaux provenant de chacune des voies d'acquisition sont analysés séparément, puis éventuellement en corrélation de façon à détecter les phénomènes de propagation (propulsif, stationnaire, rétropropulsif).

L'acquisition des données suppose la mise au point de protocoles relativement normalisés quant aux conditions de réalisation des examens, notamment en condition de durée, et d'évènements (par exemple pour l'oesophage déglutition sèche, déglutition d'eau, douleurs, perfusion d'HCL, distension de ballonnets...).

L'objectif d'une analyse de motricité digestive est d'obtenir des signaux représentatifs du déplacement des ondes mécaniques de propagation dans le circuit digestif, et de leurs caractéristiques, ainsi que l'étude des sphincters.

L'étude de la motricité digestive basée sur des mesures de manométrie, est un outil complémentaire des examens morphologiques que sont la radiologie et l'endoscopie. Outre son intérêt diagnostique, elle permet une meilleure caractérisation de la physio-pathologie et de la pharmacologie digestives.

La motricité oesophagienne permet notamment de préciser les causes des mysphalgies, des douleurs thoraciques et du reflux gastro-oesophagien. La motricité ano-rectale s'applique notamment à l'étude des phénomènes de constipation, ou d'incontinence anale, et à leur rééducation. Les motricités colique et de l'intestin grêle relèvent encore essentiellement du domaine de la recherche.

De façon connue, un examen classique s'effectue en reliant un enregistreur polygraphe en sortie des capteurs de pression dont les signaux ont été préamplifiés.

La manipulation comprend par exemple les phases suivantes :

. phases d'observation des signaux reçus des capteurs, pour valider l'appareillage,

. phase d'enregistrement en continu des signaux de motricité,

. phase d'enregistrement successivement sur n portions d'acquisition des signaux de motricité,

. phase de dépouillement des résultats.

Dans le cas d'un dépouillement manuel, il faut compter une demi-heure à trois quarts d'heure environ en manométrie de type oesophagienne et environ une après-midi en manométrie de type colique.

On connaît également des dispositifs visant à automatiser partiellement l'opération de dépouillement des résultats, comme décrits dans l'article "systèmes automatisés de traitement des signaux de motricité digestive" C. ROZE, Gastroentérol. Clin. Biol., 1988, 12 368-375.

Ainsi, un logiciel a été conçu par la Société Sensor Médics pour le traitement des signaux de manométrie oesophagienne en sortie de polygraphe. Les signaux sont stockés dans un micro-ordinateur, et peuvent être ensuite affichés à l'écran par séquences, de façon à pouvoir effectuer diverses mesures en utilisant un système de repérage sur écran à l'aide de curseurs mobiles.

Ce système connu n'a toutefois qu'un fonctionnement semi-automatique, qui est lent et délicat à manier.

On connaît également un système de traitement mis au point par le laboratoire de pharmacologie de l'INRA Toulouse (Thierry HACHET) utilisé pour la mesure de l'index moteur gastro-intestinal pendant des durées de l'ordre de 24 heures chez le chien. L'index moteur est défini comme l'aire comprise entre la courbe d'activité contractile et la ligne de base. Cet index est déterminé et stocké par tranches de 30 ou 60 minutes, sur huit pistes maximum.

Les signaux recueillis en motricité digestive, et singulièrement en manométrie digestive, présentent un certain nombre de caractéristiques qui rendent délicates les opérations d'analyse automatique. Parmi ces caractéristiques, on peut noter :

. l'apparition d'artefacts dans le signal, le plus souvent dus à des mouvements intempestifs du patient (toux,...),

. la dérive de la ligne de base niveau zéro de pression du signal obtenu, lié par exemple aux effets de paroi apparaissant entre les cathéters alimentés en perfusion et la paroi de la portion de tube digestif dans laquelle ils sont introduits.

Le système connu réalisé par HACHET comporte un système de correction de dérive de la ligne de base, au moyen d'un algorithme de reconnaissance basé sur la mesure de 150 valeurs comprises dans une fourchette de plus ou moins 2,3 % de la ligne de base précédente (cas d'un échantillonnage à une fréquence de 0,5 Hz). La nouvelle valeur calculée remplace la valeur précédente et ainsi de suite.

Toutefois, ce principe de correction de dérive de la ligne de base ne permet pas de rendre compte, de façon suffisamment précise, des phénomènes générateurs du signal, ce qui fausse les analyses quantitatives basées sur la courbe rectifiée.

On connaît par ailleurs, dans d'autres domaines, des procédés de traitement du signal proposant de calculer les surfaces formées par des impulsions dans des signaux présentant des lignes de base sujettes à dérive. Tel est par exemple le cas de l'article "A New Computing Integrator for Chromatography" (Un nouveau procédé de calcul d'intégrales appliqué à la chromatographie) du "Journal of Chromatographic Science", vol.9, n° 12, du 10.12.1971, p.710-716 de J.D HET TINGER et al.

Cet article propose une correction tangentielle de la dérive de la ligne de base d'un signal de chromatographie.

Cependant, le procédé de correction décrit, de par la spécifité de l'application effectuée, ne peut s'appliquer à un signal de manométrie colique : en effet, les signaux de chromatographie sont des signaux se présentant par salves, le nombre d'impulsions est limité et la dérive de la ligne de base est due à la dérive du détecteur des composants à analyser.

Or, dans le domaine de la manométrie colique, la dérive de la ligne de base est essentiellement d'origine physiologique, puisqu'elle résulte principalement des effets de tonus musculaire.

Les principes appliqués à l'analyse d'un signal de chromatographie ne peuvent donc s'appliquer à un signal de manométrie colique.

D'autre part, dans le domaine de l'électromyographie, c'est-à-dire de l'étude de l'activité électrique d'un organe, il peut également se présenter une dérive de la ligne de base d'un signal comme décrit dans l'article "Computer Analysis of Intestinal Motor Activity" (Analyse par ordinateur d'un signal d'électromyographie intestinale) Automedica, 1987, vol. 7, n° 4, p.221-236 de A.J. FLATT et al.

Cet article décrit notamment un procédé de correction de la dérive de la ligne de base d'un signal d'électromyographie intestinale toutes les minutes

L'analyse des signaux d'électromyographie intestinale ne permet cependant pas de recouvrir les phénomènes mesurés par manométrie colique. En effet, certaines activités électriques ne donnent aucune onde et deux excitations pouvant s'annihiler mutuellement. De plus, un muscle malade, par exemple atteint d'une sclérose de paroi, peut ne pas répondre à une excitation électrique.

Les domaines d'électromyographie intestinale et de manométrie colique sont donc, de par la spécificité des signaux relevés, fondamentalement différents, et les méthodes appliquées dans le domaine de l'électromyographie ne sont pas transposables à celui de la manométrie colique.

L'objectif de l'invention est de fournir un procédé d'analyse qui pallie ces inconvénients de l'état de la technique.

Plus précisément, un premier objectif de l'invention est de fournir un système automatisé, notamment adapté aux spécificités des signaux de manométrie digestive, telle que la manométrie colique, visant à permettre la réduction du temps de dépouillement des résultats, et l'obtention de fonctions performantes de représentation graphique, d'analyse et d'archivage des données.

Un autre objectif essentiel de l'invention est de fournir un tel système qui permette de rendre compte de façon plus fidèle des phénomènes générateurs du signal analysé, notamment au moyen d'une succession adéquate d'opérations de traitement spécifiquement adaptées. Ces opérations doivent notamment permettre de discriminer, dans le signal d'origine, les caractéristiques significatives pour le traitement et l'obtention des résultats.

Un objectif complémentaire est de fournir un procédé permettant une mise en oeuvre aisée et rapide, notamment au moyen d'une compression de données adéquate.

Ces objectifs, ainsi que d'autres qui apparaîtont par la suite, sont atteints à l'aide d'un procédé d'analyse d'un signal du type représentable par une courbe portant des trains d'ondulations et présentant une dérive de la ligne de base, notamment pour l'analyse d'un signal de manométrie digestive, par exemple en manométrie colique,

procédé caractérisé en ce qu'il comprend une première étape de correction de la dérive de ladite ligne de base, consistant à identifier des minima significatifs dans ladite courbe à trains d'ondulations parmi des points candidats, et à réaligner ladite courbe en relation avec lesdits minima significatifs identifiés, et une seconde étape d'analyse quantitative de ladite courbe ainsi réalignée,

et en ce que ladite étape d'identification des minima significatifs consiste à identifier itérativement chaque nouveau minimum significatif par explorations successives de points de ladite courbe suivant le dernier minimum significatif identifié, un nouveau minimum significatif étant identifié en prenant en compte, pour chaque point candidat courant exploré, au moins un des points candidats précédents et/ou suivants, et en prenant ou non une décision de sélection, en tant que nouveau minimum significatif, dudit point courant exploré et/ou d'un desdits points candidats précédents ou suivants en comparant les pentes des droites reliant le dernier minimum significatif identifié à chacun desdits points candidats pris en compte et au point courant exploré entre elles et avec la pente reliant les deux derniers minima significatifs identifiés.

Dans un mode de réalisation avantageux de l'invention, ladite étape d'identification des minima significatifs consiste à identifier itérativement chaque nouveau minimum significatif par explorations successives de points de la courbe suivant le dernier minimum significatif identifié, un nouveau minimum significatif étant identifié en fonction de la valeur des pentes de droite de liaison $P_1$, $P_2$, $P_3$ et $P_4$, $P_1$ étant défini comme étant la pente de la droite de liaison entre les deux derniers minima significatifs identifiés $P_2$ la pente de la droite de liaison du dernier minimum significatif au point précédemment exploré, $P_3$ la pente de la droite de liaison du dernier minimum significatif au point courant exploré et $P_4$ la pente de la droite de liaison du dernier minimum significatif au point suivant exploré.

Selon une autre caractéristique avantageuse de l'invention, ladite étape de sélection des minima significatifs est précédée d'une étape de sélection de minima candidats dans la courbe, consistant par exemple à découper la courbe en tronçons successifs ou en plages de 50 à 100 points, à y détecter des minima locaux, et à retenir au moins un point d'ordonnée minimale dans une fenêtre autour d'une moyenne récursive pour chacun desdits tronçons.

Selon une autre caractéristique essentielle de l'invention, le nouveau minimum significatif est choisi en fonction de la durée d'un tonus musculaire. Dans ce cas, le minimum significatif est avantageusement choisi, sous certaines conditions, comme étant le premier minimum candidat sélectionné au-delà de la durée d'un tonus musculaire, lorsqu'aucun nouveau minimum significatif n'a été identifié pendant ladite durée d'un tonus musculaire, à partir du dernier minimum significatif identifié.

On notera que cette caractéristique de l'invention est fondamentale, puisqu'elle permet d'éviter d'absorber, lors de l'analyse de la courbe, les phénomènes de tonus musculaire. Une correction maladroite de la ligne de base peut en effet conduire à fonctionner sous la forme d'un filtre passe-haut absorbant l'onde de tonus qui est d'une durée comprise entre environ 5 et environ 15 minutes, et généralement aux alentours de 10 minutes. L'absorption de ce tonus fausse ensuite les résultats et notamment le calcul de l'index moteur.

Selon l'invention, l'initialisation de l'étape d'identification des minima significatifs consiste à sélectionner comme première ligne de base la droite réunissant les deux premiers minima candidats sélectionnés et/ou comme première valeur de la moyenne récursive, la valeur du minimum candidat sélectionné sur la première plage de points explorée. Ce processus d'initialisation provoque une période transitoire de mise en route du traitement, qui converge rapidement vers un régime stable de correction de la ligne de base.

Avantageusement, ladite étape de correction de la dérive de la ligne de base est précédée d'une étape de filtrage, comprenant une opération d'élimination des artefacts de la courbe et/ou une opération de filtrage à fenêtre.

Selon une autre caractéristique de l'invention, ladite opération d'élimination des artefacts consiste à effectuer pour chaque point de la courbe, un calcul de la pente de la droite le liant aux points suivants de la courbe, et à initialiser le traitement d'élimination d'artefacts en cas de pente supérieure à un seuil prédéterminé, ledit traitement d'élimination des artefacts consistant à éliminer tous les points de la courbe suivant le point d'initialisation et dont la droite les liant auxdits points d'initialisation présente une pente supérieure audit seuil prédéterminé.

Préférentiellement, ladite étape de correction de la dérive de la ligne de base est suivie d'une détection des zones d'activités, dans la courbe, comprenant une opération de détection des points de la courbe réalignée d'ordonnée inférieure à un seuil prédéterminé pour la détermination des zones de début et de fin d'activité.

Dans ce cas, ladite étape de détection des zones d'activité comprend avantageusement une opération de détermination de l'abcisse précise de début et de fin respectivement desdites zones de début et de fin d'activité, par seuillage des points environnants au moyen d'un second seuil prédéterminé, inférieur audit seuil de détermination des zones.

Ladite étape de détection des zones d'activité est préférentiellement suivie par une étape d'approximation linéaire des zones d'activité.

Avantageusement, les seuils utilisés sont ajustables par l'utilisateur.

D'autres caractéristiques et avantages de l'invention apparaîtront à la lecture de la description suivante d'un mode de réalisation préférentiel du procédé de l'invention, donnée à titre illustratif et non limitatif, et des dessins annexés dans lesquels

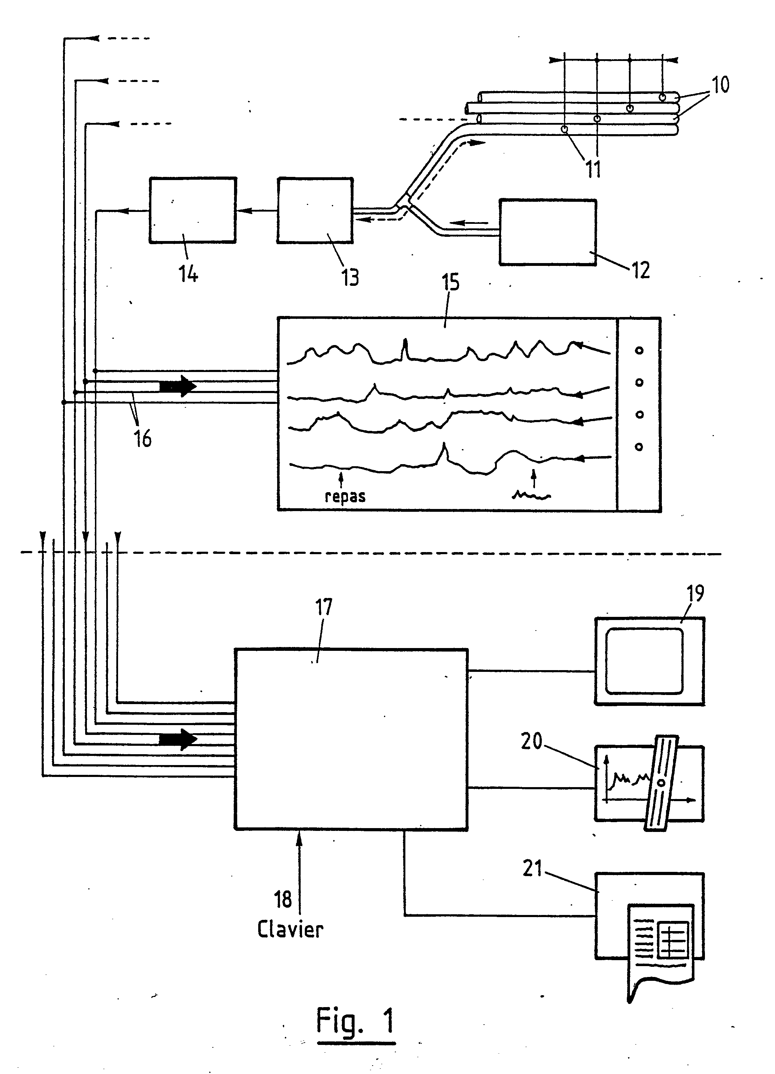

. la figure 1 est un schéma synoptique illustrant un mode d'utilisation d'une chaîne d'acquisition et de traitement de signaux en manométrie digestive, selon l'invention ;

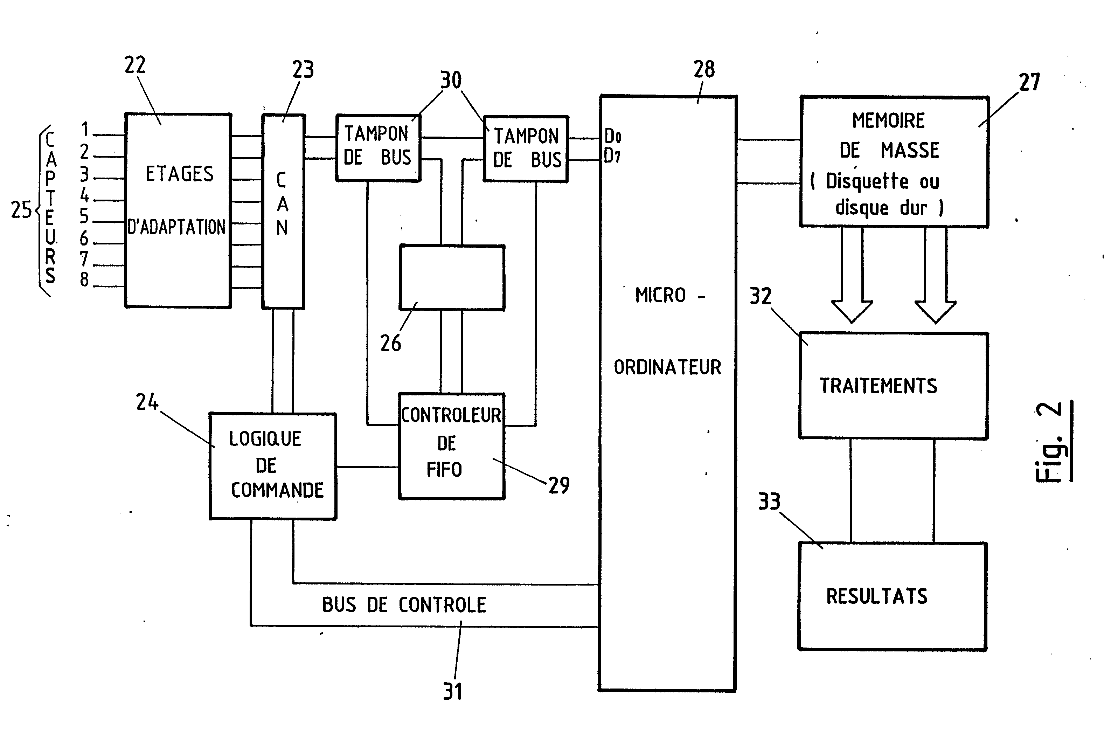

. la figure 2 est un schéma fonctionnel présentant les modules principaux d'une chaîne d'acquisition compatible avec le système d'analyse de l'invention .

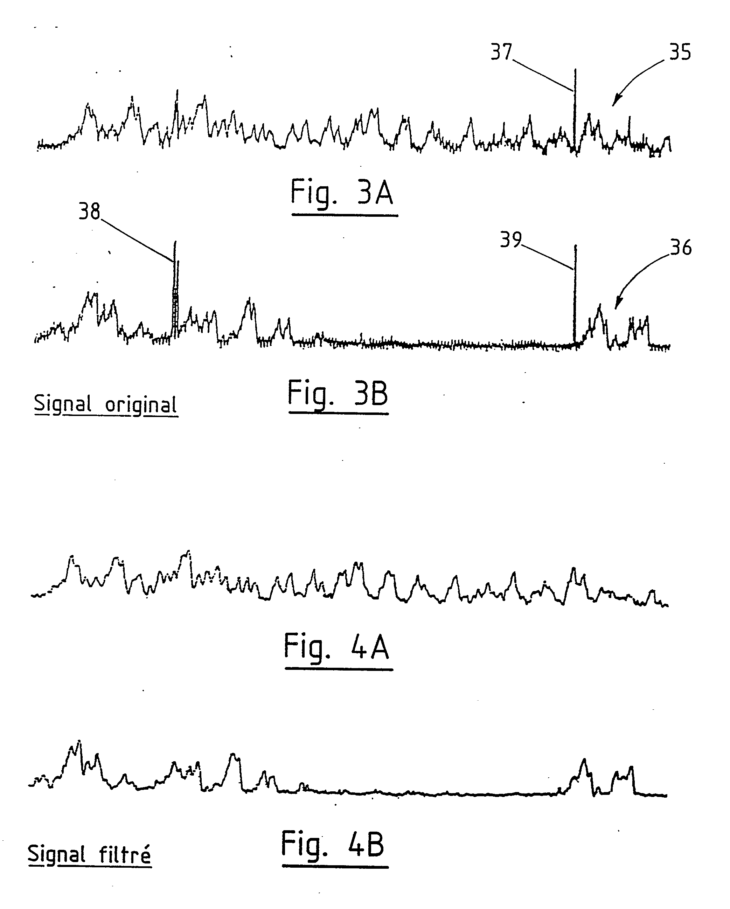

. les figures 3A-3B, 4A-4B, 5A-5B, et 6A-6B représentent des paires de forme d'onde correspondant respectivement au signal original en sortie des capteurs de pression, aux mêmes signaux après filtrage, aux mêmes signaux après alignement, et aux mêmes signaux après identification des zones d'activité et linéarisation ;

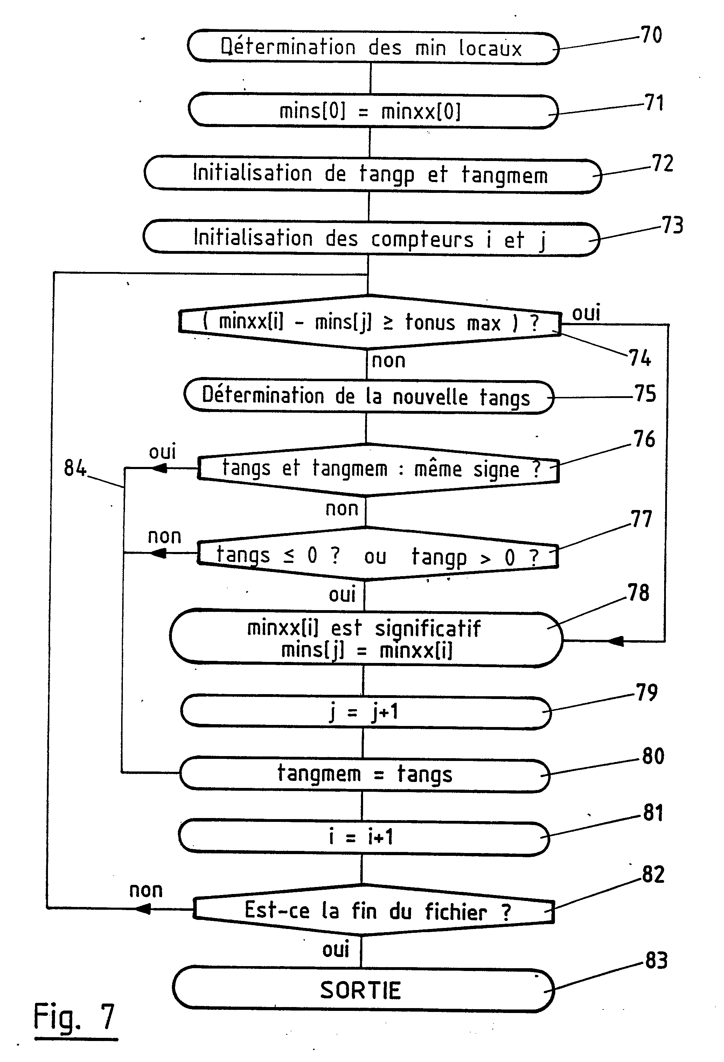

. la figure 7 est un organigramme schématisant un mode de réalisation avantageux du processus d'identification des minima significatifs, pour l'étape de correction de la ligne de base, selon l'invention ;

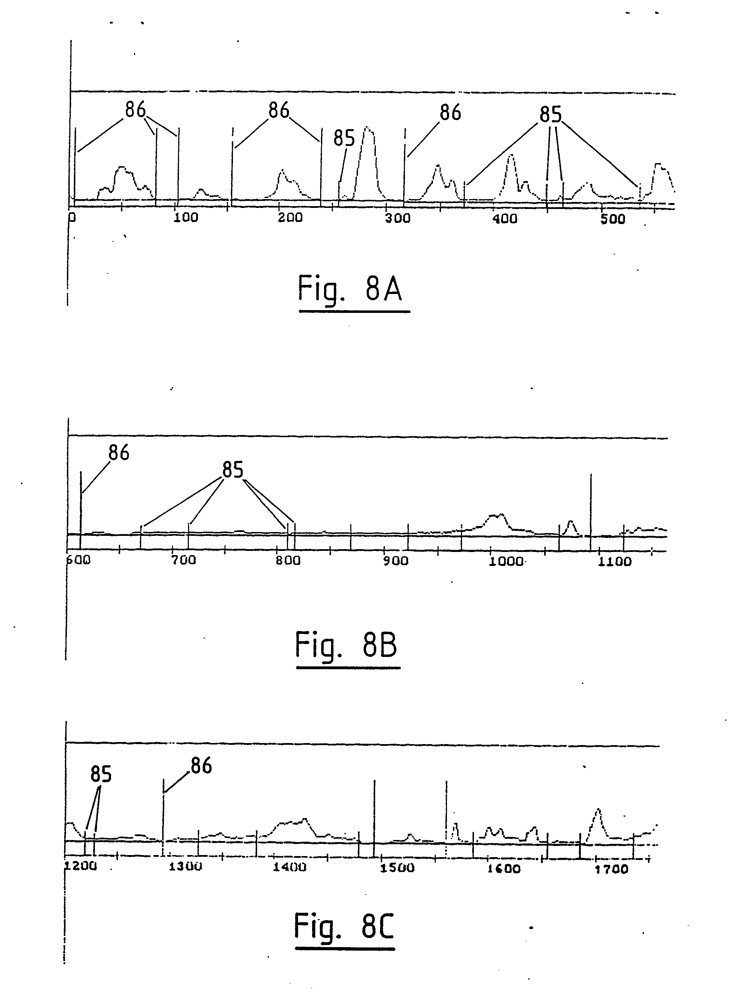

. les figures 8A, 8B, 8C illustrent la mise en oeuvre de l'étape de correction de la ligne de base suivant l'invention, par identification de minima significatifs au sein d'un ensemble de minima candidats ;

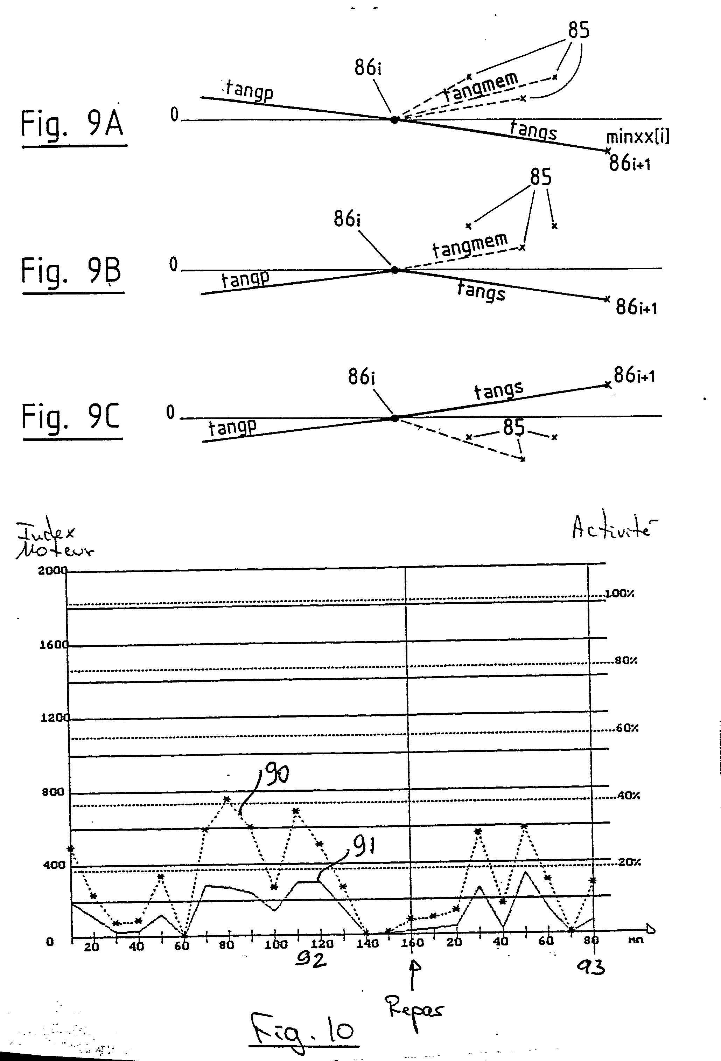

. les figures 9A, 9B, 9C schématisent les cas de branchement sélectif de l'organigramme de la figure 7, conduisant à l'identification de minima significatifs par des opérations d'analyse de pentes de droites de liaison entre les points explorés dans la courbe;

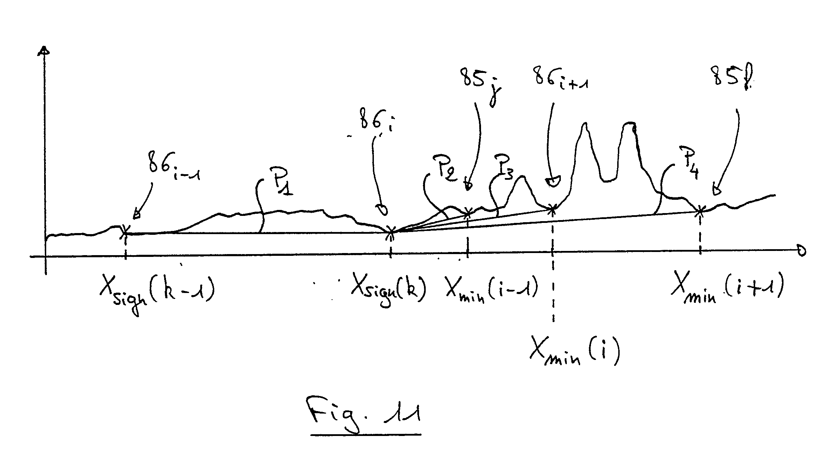

. la figure 10 est une courbe schématisant une présentation des résultats, de l'index moteur et de l'activité relevée au cours de l'analyse de signaux de manométrie colique selon l'invention.

. la figure 11 représente un signal de manométrie colique présentant une dérive de la ligne de base ainsi que quatre pentes de droite de liaison entre les points explorés dans la courbe, selon un autre mode préférentiel de mise en oeuvre de l'invention.

Le mode de réalisation décrit ici concerne essentiellement l'acquisition et le traitement de signaux de manométrie colique.

Bien entendu, les principes exposés ici sont transposables aux autres signaux de manométrie digestive, ainsi que d'une manière générale à des signaux du type constitué d'une courbe portant des trains d'ondulation de même type et présentant une dérive analogue de la ligne de base.

Comme représenté en figure 1, un mode de réalisation avantageux de la chaîne d'acquisition et de traitement comprend successivement un jeu de tubes perfusés 10 munis d'orifices étagés 11 par exemple de 5 cm en 5 cm. Chacun de ces tubes 10 est relié d'une part à un perfuseur 12 et d'autre part à un capteur de pression 13 comprenant par exemple des jauges de contraintes. Le signal de sortie du capteur de pression 13 alimente un préamplificateur 14.

De façon connue, le signal de sortie du préamplificateur 14 peut être utilisé pour le traçage des courbes représentatives de l'activité de motricité digestive, sur un enregistreur polygraphe 15. L'enregistreur comporte autant de voies d'entrée 16 que de capteurs, et donc de tubes perfusés 10.

Selon l'invention, les signaux préamplifiés obtenus en sortie du préamplificateur 14 sont acheminés vers une unité 17 d'enregistrement numérique, et d'exploitation automatique.

L'unité 17 réalise d'une part l'opération d'acquisition des signaux provenant des capteurs. Cette opération d'acquisition s'effectue avantageusement de façon interactive, au moyen d'un clavier de commande 18 permettant d'une part de fournir à l'appareil les paramètres d'acquisition de traitement, et d'autre part d'assurer la saisie d'informations complémentaires à l'acquisition des signaux captés (tels que les conditions de l'analyse : apparition de douleurs, prise de repas,...).

L'unité 17 comprend d'autre part des moyens de traitement des signaux enregistrés sous forme de données numériques, les résultats de traitement pouvant par exemple être sortis vers des terminaux tels qu'un écran de visualisation 19, des moyens de traçage de courbes 20, et des moyens 21 d'impression de résultats qualitatifs et/ou chiffrés.

Le système d'acquisition schématisé en figure 2 consiste en un ensemble de deux cartes compatibles PC XT, insérées dans un micro ordinateur 28.

Les signaux prélevés en sortie des préamplificateurs (par exemple de type BAROVAR (marque déposée)) attaquent, après adaptation 22 en amplitude (0 à 1 V correspondant à 0 à 100 mm Hg), les entrées du convertisseur analogique -numérique 23.

La logique de commande 24 associée et reliée au micro ordinateur 28 par un bus de contrôle 31 permet un échantillonage systématique des 8 voies d'entrée, à une fréquence programmable par l'utilisateur (entre 0,5 et 10 Hz), le temps de conversion du CAN 23 (80 μs) assurant une simultanéité quasi-parfaite du prélèvement des échantillons sur les voies d'entrée 25. L'utilisation d'une mémoire tampon 26 (de type FIFO) de taille suffisante, contrôlée par un contrôleur de FIFO 29 en coopération avec deux tampons de bus 30, permet d'assurer les transferts de la mémoire système 26, vers la mémoire de masse 27 (disquette ou disque dur) d'un micro-ordinateur 28 sans perte d'information. Le mode de fonctionnement retenu, comporte une initialisation du nombre de voies utilisées 25 et de la fréquence d'échantillonnage, puis le lancement d'une séquence d'enregistrement.

Le programme d'acquisition permet avantageusement d'interrompre une séquence d'enregistrement et d'en lancer une autre à volonté.

Les communications, en phase d'acquisition, entre les cartes d'acquisition et le micro ordinateur 28 sont gérées au travers des ports et interruptions de celui-ci (Interruptions matérielles). Ces interruptions interviennent lorsque la mémoire FIFO 26 est pleine pour déclencher un transfert de la mémoire 26 vers la mémoire centrale du micro ordinateur 28, ou, au cours de ce transfert, pour répondre à toute demande de mémorisation de données émanant du convertisseur 23.

Ainsi, on peut aussi bien envisager:
. soit une procédure de vidage de la mémoire 26 lorsqu'elle est pleine, procédure acceptable quand la fréquence d'échantillonnage est faible (par exemple 10 Hz maximum) ce qui autorise un vidage complet de la mémoire 26 entre l'acquisition de deux échantillons consécutifs,
. soit une procédure de vidage systématique de la mémoire, tant qu'aucune interruption en écriture n'est émise par les moyens d'acquisition. Cette procédure convient par exemple pour la récupération en mode accéléré de signaux enregistrés sur bande magnétique.

Le logiciel d'acquisition permet de sauvegarder le contexte de la saisie, à savoir:
heure et date de début et de fin de chaque séquence d'enregistrement, fréquence d'échantillonnage, chronologie des événements extérieurs (ex : début et fin de douleurs ressenties en cours d'examen ...), informations concernant le patient (identité, âge,...), et tout autre message utile pour l'exploitation ultérieure.

Les données enregistrées sur disquette ou disque dur 27 sont exploitables par tout autre micro ordinateur compatible, au cours d'une étape de traitement 32 permettant d'obtenir des résultats 33 destinés à l'opérateur.

Selon l'invention, on réalise avantageusement en parallèle l'acquisition des données manométriques avec des données de contexte, entrées par exemple au clavier 18 (Fig 1), telles que les indications de prise de repas, de douleurs, de prise de médicaments, de manoeuvres spécifiques (gonflage de ballonnets, avec dimension, position et durée,...) au moment de leur survenance. Ces indications seront prises en compte et restituées lors du traitement et de l'émission des résultats 33.

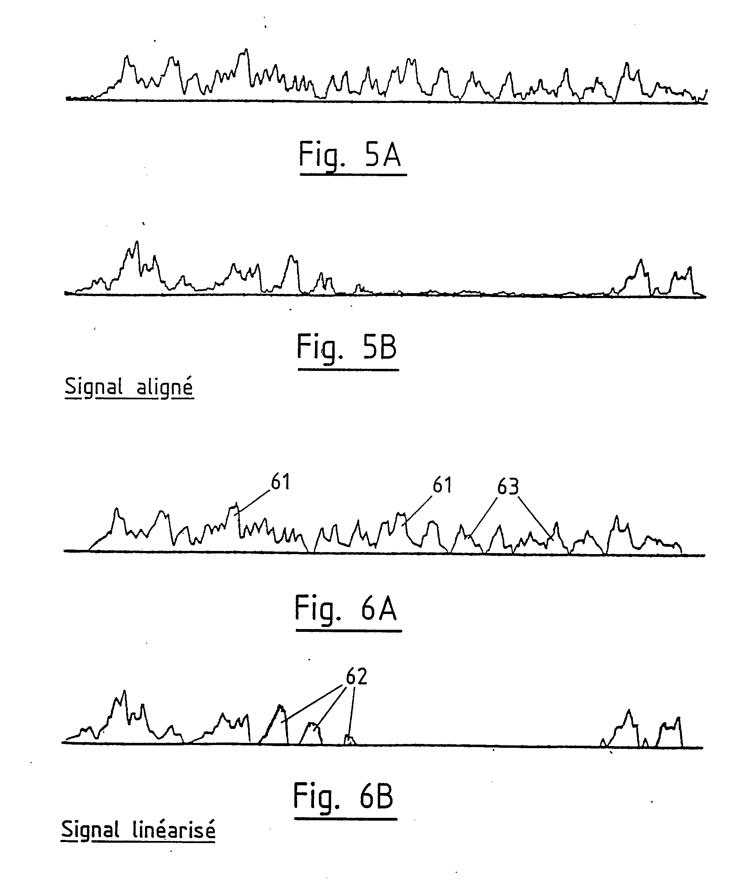

La procédure de traitement des données stockées est illustrée par les Fig 3A,3B à 6A,6B. Le traitement du signal original (Fig 3A, 3B) comprend avantageusement les étapes suivantes :
. Filtrage du signal numérisé (Fig 4A, 4B)
. Recherche de la ligne de base (Fig 5A, 5B et Fig 8A, 8B, 8C)
. Détection des zones d'activité et linéarisation (Fig 6A, 6B).

Le signal d'origine est représenté, à titre d'exemple, sous la forme de deux portions de courbes 35, 36, correspondant à deux périodes quelconques distinctes d'acquisition. Les mêmes courbes 35, 36 se retrouvent, telles qu'elles apparaissent après les traitements successifs, en Figures 4A, 5A, 6A, et 4B, 5B, 6B respectivement.

Le filtrage du signal numérisé dont les effets sont schématisés en Fig 4A, 4B, se compose de deux parties :
. Il élimine les artefacts 37, 38, 39 en se basant sur le calcul de la dérivée filtrée. Partant d'un point d'indice n on incrémente i tant que $\mid Y(n+i)-Y(n) \mid / i >$ seuil. Une interpolation linéaire entre les points d'indice n et n+i permet alors d'éliminer les artefacts dus à des parasites extérieurs ou à des mouvements brusques du patient au cours de l'examen.
. Il élimine les ondes respiratoires par utilisation d'un filtre médian de largeur de fenêtre 5 points. Ce traitement présente les propriétés intéressantes d'éliminer les bruits de type impulsionnel dont l'étendue spatiale est inférieure à la moitié de la fenêtre (cas des perturbations liées à la respiration), tout en préservant les transitions du signal en rampe et en échelon. C'est un filtre non linéaire, facilement implantable et parmi ceux nécessitant les temps de calcul les plus courts.

D'une manière générale, un filtrage à fenêtre s'exprime par
$$m_2 = (1-1/N)m_1 + 1/N \, dn(i)$$
avec : $m_1$ = moyenne précédente (initiale)
$m_2$ = moyenne courante (nouvelle moyenne)
$N$ = taille de la fenêtre
$dn(i)$ = dernier échantillon retenu.

A titre d'exemple, une loi de filtrage pourrait s'exprimer, pour une fréquence d'échantillonnage de 3 Hz, par : $m_2 = m_1 + (m_1 + x_{min})C$
avec . $N > 1820$ (par exemple $N = 2048$) pour prendre une valeur supérieure à la durée d'un tonus (voir plus loin)
. $x_{min}$ = minimum candidat (voir plus loin)

A la place d'un filtre médian, il est possible d'utiliser un filtre numérique passe-bas en fixant le seuil de filtrage en fonction de la nature du signal à traiter. En l'occurrence, pour l'analyse du signal de manométrie colique étudié, on prévoit une bande atténuée entre 0,15 et 0,25 Hz pour le filtre passe-bas.

La recherche de la ligne de base correspond à l'étape la plus délicate à mettre au point du fait de la complexité du signal de manométrie colique (périodes d'activité irrégulières).

En manométrie colique, on prend en compte trois types d'ondes distinctes, à savoir:
. Monophasiques (1 seul pic),
. Polyphasiques (au moins 2 pics),

. "Onde(s) + Tonus", le(s) onde(s) étant de l'un ou l'autre des types ci-dessus.

La durée maximale d'une "onde + Tonus" est d'environ 10 min (soit 1800 points pour une fréquence d'échantillonnage de 3 Hz). Un tonus correspond à une ondulation basse fréquence (période 10 min environ) modulée par les ondes mono ou polyphasiques.

La ligne de base doit respecter la forme des ondes, et donc ne pas absorber ni les ondes mono ou polyphasiques, ni les ondulations de tonus.

La récupération des données (après élimination des artefacts et filtrage médian) se fait par plages de 6144 points ( soit 6ko), mais le déplacement sur le fichier est de 4096 (soit 4Ko). La zone excédentaire de 2Ko permet un recouvrement, entre deux traitements consécutifs, d'une durée supérieure à la période d'un tonus.

L'organigramme de la figure 7 présente le détail d'un exemple de la procédure de correction de la ligne de base.

Comme on le voit dans cet exemple, les étapes essentielles du procédé de l'invention sont les suivantes :
. Détermination de minima locaux dans la courbe (étape 70) ;
. Initialisation des variables (étapes 71, 72, 73) ;
. Exploration itérative des minima locaux sélectionnés, de façon à identifier les minima significatifs, par tests et branchements (étapes 74 à 82), jusqu'à traitement de la totalité du fichier (étape 83).

Pour chaque plage de 6144 points, les minima locaux sont déterminés de la manière suivante :
. Dans chaque tranche de 50 points ou de 100 points (par exemple), on extrait l'échantillon ayant la plus petite ordonnée et se trouvant dans une fenêtre autour de la moyenne récursive, par exemple tous les points inférieurs à la valeur de la moyenne récursive augmentée d'un faible seuil (par exemple aux alentours de 8 mm Hg). Cette dernière est réactualisée après l'extraction d'un minimum local. L'abcisse et l'ordonnée du minimum local courant sont respectivement notées : minxx[i] et a[minxx[i]]. Le nombre de points pris résulte d'une optimisation d'un compromis entre les temps de calcul et la précision de la ligne de base calculée.

On a représenté en figures 8A, 8B, 8C, l'étape de sélection des minima locaux, candidats à la sélection des minima significatifs, par des traits verticaux 85, 86, de deux tailles différentes :
. Les traits verticaux 85 de taille courte correspondent à des minima locaux qui n'ont pas été sélectionnés comme minima significatifs ;
. Les traits de grande taille 86 correspondent à des minima locaux qui ont été sélectionnés comme minima significatifs.

Les figures 8A, 8B, 8C correspondent à trois portions consécutives de signaux numérisés, et filtrés, sur les plages 0 à 600 points, 600 à 1200 points et 1200 à 1800 points respectivement.

La détermination des minima significatifs, parmi l'ensemble des minima locaux candidats déterminés, s'effectue dans les conditions suivantes :
a) La procédure précédente, qui détecte les minima locaux permet de remplir le tableau suivant : (minxx[0],minxx[1],......, minxx[hmax])
hmax étant le nombre de minima locaux extraits dans une plage de 6144 points.

Le calcul des minima significatifs se fera à partir de ce tableau des minxx.

Dans un premier mode de réalisation, ladite étape d'identification des minima significatifs consiste à identifier itérativement chaque nouveau minimum significatif 86 par explorations successives des minima candidats 85 de la courbe suivant le dernier minimum significatif identifié,
un nouveau minimum significatif 86 étant identifié en fonction de la valeur des pentes de droites de liaison tangmem et tangs, tangmem étant défini comme la droite de liaison du dernier minimum significatif au point précédent exploré, et tangs la droite de liaison du minimum significatif au point courant exploré.

Avantageusement, ceci est réalisé en mettant en oeuvre au moins un des deux processus suivants (étapes 76 et 77) :
. un nouveau minimum significatif est identifié lorsque tangs et tangmem sont de signes contraires, et tangs est négative ou nulle.
. un nouveau minimum significatif est identifié lorsque tangs et tangmem sont de signes contraires, et que la droite de liaison des deux derniers minima significatifs tangp est strictement positive.

Selon une autre caractéristique essentielle de l'invention, le nouveau minimum significatif est choisi en fonction de la durée du tonus musculaire. Dans ce cas, le minimum significatif est avantageusement choisi comme étant le premier minimum candidat 85 sélectionné au-delà de la durée d'un tonus musculaire, lorsqu'aucun nouveau minimum significatif 86 n'a été identifié pendant ladite durée d'un tonus musculaire, à partir du dernier minimum significatif 86 identifié (étape 74).
b) Définition des tangentes utilisées dans le programme :

(1) tangp = (a[mins[j]] - a[mins[j-1]] / (mins[j] - mins[j-1]) mins[j] étant l'abcisse du minimum significatif courant (le dernier min significatif qui a été retenu).
tangp est la tangente entre le minimum significatif courant et le minimum significatif du précédent.

(2) tangs = (a[minxx[i]] - a[mins[j]]) / (minxx[i] - mins[j]) est la tangente entre le minimum local courant et le minimum significatif courant.

(3) tangmem = tangs (étape 80)

Cette relation permet de mémoriser la valeur précédente de tangs.

(4) tangp et tangmem sont initialisés au début de chaque nouvelle plage, en fonction des valeurs finales de la plage précédente (étape 72).

Pour estimer qu'un minimum est significatif, l'une des conditions suivantes doit être satisfaite :
**Si** la distance entre le minxx[i], minimum local, et le mins[j], minimum significatif courant, est supérieure ou égale à 1800 points,
**alors** : minxx[i] est significatif.
**Si** tangs et tangmem ont le même signe
**alors** : minxx[i] n'est pas significatif (84).
**Si** tangs et tangmem n'ont pas le même signe
**alors** : minxx[i] peut être significatif si au moins l'une des deux conditions suivantes est vérifiée (étape 77)

a) tangs $< = 0$, pour n'importe quel signe de tangp

b) tangp $>0$ , pour n'importe quel signe de tangs.

Les trois solutions correspondant à ces cas d'identification d'un minimum significatif $86_{i+1}$ à partir des valeurs des tangp, tangmem et tangs et du minimum significatif précédemment sélectionné $86_i$ sont schématisées en Fig. 9A, 9B, 9C.

Dans tous les cas, on vérifie que tangs et tangmem ne sont pas de même signe.

La Fig 9A illustre le cas où tangp $< = 0$ et tangs $< = 0$

La Fig 9B illustre le cas où tangp $>0$ et tangs $< = 0$

La Fig 9C illustre le cas où tangp $>0$ et tangs $>0$.

Dans une version améliorée de cette méthode de détermination de minima significatifs par comparaisons de tangentes, on peut appliquer le processus illustré en Fig.11.

La figure 11 représente un signal de manométrie colique auquel est appliquée une méthode de détection de minima significatifs prenant en compte de façon plus précise l'environnement de chaque point courant exploré, d'une part en augmentant le nombre de points pris en compte autour du point courant exploré, (au moins un point candidat précédent et au moins un point candidat suivant) et, d'autre part, en affinant les conditions de sélection d'un minimum significatif par la méthode de comparaison de pentes.

Cette méthode constitue un mode de mise en oeuvre préférentiel de la présente invention.

Après une détection des minima candidats $(X_{min}, Y_{min})$ par plages de 50 ou 100 points, une validation de ceux voisins de la moyenne récursive, calculée sur tous les minima précédemment retenus, est effectuée.

Les minima obtenus subissent alors une opération de tri consistant à ne conserver qu'un certain nombre d'entre eux qui constitueront les minima significatifs définissant la ligne de base.

Le tri est effectué à la fréquence d'échantillonnage de 3 Hz pour un ensemble de 1800 points, afin de respecter les durées maximales des tonus musculaires.

Le processus représenté à la figure 11 prend en compte cinq minima, à savoir les deux derniers minima significatifs retenus (définissant la ligne de base courante) et un minima candidat $(85_j, 85_l)$ de chaque côté du minimum candidat courant exploré $86_{i+1}$

La méthode de sélection de chaque nouveau minimum significatif repose sur un jeu d'opérations de comparaisons de quatre pentes $P_1$ à $P_4$, définies par :

$P_1 = [Y_{sign}(k) - Y_{sign}(k-1)]/ [X_{sign}(k) - X_{sign}(k-1)]$, pente de la ligne de base en cours,

$P_2 = [Y_{min}(i-1) - Y_{sign}(k)]/ [X_{min}(i-1) - X_{sign}(k)]$, pente entre le dernier minimum significatif et le minimum local i-1,

$P_3 = [Y_{min}(i) - Y_{sign}(k)]/ [X_{min}(i) - X_{sign}(k)]$, pente entre le dernier minimum significatif et le minimum local courant i,

$P_4 = [Y_{min}(i+1) - Y_{sign}(k)]/ [X_{min}(i+1) - X_{sign}(k)]$ relatif à i+1 où :

- $(X_{sign}(k), Y_{sign}(k))$ et $(X_{sign}(k-1), Y_{sign}(k-1))$ désignent respectivement les coordonnées des dernier (k) et avant dernier (k-1) minima significatifs retenus.

- $X_{min}(i), Y_{min}(i)$ sont relatifs au minimum local courant i,

- $X_{min}(i-1), Y_{min}(i-1)$ se rapportent au minima local i-1,

- $X_{min}(i+1), Y_{min}(i+1)$ se rapportent au minima local i+1.

Un minimum local, d'indice i ou i-1, est retenu comme étant significatif s'il vérifie l'une des conditions respectives suivantes :

- signe $P_2$ différent du signe $P_3$
si $P_1 \geqq 0$ et $P_3 > 0$
**alors** $X_{sign}(k+1) = X_{min}(i-1)$
**sinon**
si $P_1 \leqq 0$ et $P_3 < 0$ et $P_4 > P_3$
**alors** $X_{sign}(k+1) = X_{min}(i)$
- sinon si signe $P_2$ = signe $P_3$
si $(P_2 < 0$ et $P_3 < 0)$ et $(P_3 > P_2)$
**alors** $X_{sign}(k+1) = X_{min}(i-l)$
si $(P_2 > 0$ et $P_3 > 0)$ et $(P_3 < P_2)$ et $[(P_4 > P_3)$
**ou** durée tonus max $(X_{min}(i) - X_{sign}(k) > 1800]$
**alors** $X_{sign}(k+1) = X_{min}(i)$

L'homme du métier notera que ces expressions conditionnelles permettent de prendre en compte des cas pathologiques où le tonus est supérieur à 10 mn (1800 points). Dans le cas où on décide le recollage forcé de la ligne de base, il est alors avantageux de sélectionner comme minimum significatif, non pas le minimum courant, mais un des minima de la plage courante explorée de la courbe. Ce minimum est par exemple choisi comme présentant la pente la plus faible pour la droite reliant ledit minimum au dernier minimum significatif.

L'alignement du signal est réalisé en faisant la différence entre le signal filtré et la ligne de base obtenue par interpolation linéaire entre les minima significatifs.

L'utilisation de cinq points et quatre pentes pour la détermination de la ligne de base permet d'obtenir de meilleurs résultats à analyser que ceux obtenus par la méthode utilisant trois tangentes précédemment décrite.

D'autres variantes de la méthode générale de l'invention pourront être mises en oeuvre par l'homme du métier, suivant le nombre de minima candidats que l'on prend en compte de part et/ou d'autre du point courant exploré, la faculté ou non de retenir comme minimum significatif soit le point courant, soit un autre des candidats pris en compte, et la nature des tests réalisés sur les pentes des droites de liaison des minima.

Dans l'une ou l'autre version, l'initialisation de ladite étape d'identification desdits minima significatifs consiste, après l'initialisation de la moyenne, à sélectionner comme première ligne de base, la droite réunissant les 2 premiers minima candidats sélectionnés. L'initialisation de la moyenne revient à prendre pour première valeur de moyenne, la valeur du minimum candidat sélectionné sur la première plage de points explorée.

L'étape de correction de la ligne de base est suivie d'une étape de détection des zones d'activité, qui repose sur l'initialisation de deux seuils de détection d'amplitude, ajustables par l'utilisateur, l'un assez élevé S1 (toute activité inférieure à S1 sera exclue de toute analyse ultérieure) pour une bonne efficacité de détection, l'autre plus bas S2 (par exemple S2 = S1/3) pour l'estimation des abscisses du début et de la fin de chaque période d'activité et le calcul de sa durée. La courbe, bien qu'inférieure au seuil S2, peut ne pas retourner à zéro. Tout échantillon n tel que Yn < S2 est mis à zéro. Avantageusement, le seuil S1 correspond par exemple à 1/25 environ de la dynamique totale du signal, ce qui correspond dans une des applications testées à approximativement 4 mm Hg.

Enfin, l'approximation linéaire des zones d'activité associe au signal filtré et aligné une série temporelle définie par une suite de segments adjacents. Ceci permet la compression des données et l'élimination des bruits résiduels subsistant après filtrage.

L'appartenance du point courant n au segment "en cours" k est décidée après considération d'un critère de pente.

Calcul de la pente : pente(n) = (Yn-Yk) / (Xn-Xk)

avec Xk, Yk les coordonnées de l'origine du segment k

et Xn, Yn les coordonnées du point courant.

Partant d'une pente pour le segment "en cours" (pente (k)) on se fixe une plage de recherche : pente (k) + σ à

pente (k) - σ .

Si la pente (n) n'appartient pas à cette plage, le point courant n'appartient pas au segment initial (indice k). Il est dit significatif (initialisation d'un nouveau segment k + 1 dont l'origine sera X(n-1), Y(n-1)) sinon on passe au point suivant (suite du segment "en cours" k).

Les étapes de détection des zones d'activité et de linéarisation sont conçues pour permettre le respect des formes d'ondes et la conservation des extréma (valeurs et positions des pics modifiées au minimum).

Les Fig 6A, 6B fournissent l'état des courbes après la succession de traitements.

Un classement des ondes est effectué qui consiste en la séparation des divers types d'ondes constitutives du signal à savoir :

. ondes monophasiques 62 (1 seul pic)

. ondes polyphasiques 63 (au moins deux pics et une durée inférieure à un seuil de durée Sd).

."ondes + tonus "61 (ondes de durée supérieure ou égale au seuil Sd).

Pour chaque type d'onde de nombreux paramètres peuvent être extraits dont les plus importants sont:

. le nombre de monotonies (nombre de segments obtenus lors de la linéarisation de l'onde)

. la durée Ti = Tfin - Tdébut

. la valeur de crête

. l'amplitude moyenne Am = (Si/Ti) (Si étant la surface de l'onde, Ti sa durée)

. la fréquence des ondes élémentaires superposées aux ondes polyphasi ques.

Pour chaque plage du signal (typiquement 10 minutes, mais cette durée est réglable par l'utilisateur) les caractéristiques suivantes sont déterminées :

. pourcentage d'activité pour chaque type d'ondes ( des durées des ondes d'un type donné / durée de la plage)

. pourcentage d'activité totale pour une plage

. index moteur (amplitude moyenne x pourcentage d'activité totale pour une plage).

Pour chaque enregistrement les paramètres retenus sont :

. activité totale

. index moteur.

Des représentations graphiques en fonction du temps, histogrammes et impression des résultats sous forme de tableaux peuvent être extraits, comme représenté en Fig. 10.

Dans l'exemple représenté, la courbe 90 correspond par exemple à l'activité globale calculée, et la courbe 91 a la valeur de l'index moteur, sur une période de 240 minutes. Deux séquences distinctes consécutives sont repérées, à savoir une période de type basale 92, suivie d'une période post-prandiale 93 déclenchée par la prise d'un repas.

Le procédé selon l'invention permet ainsi de fournir au praticien chargé d'analyser les signaux de manométrie colique mesurés sur un patient, les signaux vraiment représentatifs qu'il aurait dû, sans la mise en oeuvre du procédé, isoler et analyser "manuellement".

Les praticiens disposent donc d'informations nouvelles et d'un enrichissement du contexte de l'examen :
- statistiques sous formes chiffrées et graphiques du contenu des signaux enregistrés : .durées - amplitudes maximales, moyennes...) des ondes en fonction de la période;
- comparaison entre les voies et évolution dans le temps ;
- enregistrement et visualisation sur les restitutions graphiques des douleurs ressenties par le patient...

Le résultat en est la constitution d'un dossier contenant l'ensemble des informations utiles et consultable par le praticien pour l'établissement de son diagnostic.

Bien entendu, la présente invention n'est pas limitée aux caractéristiques et applications ci-décrites, et l'homme du métier imaginera aisément d'autres applications sans pour autant sortir du cadre de l'invention.

**Revendications**

1. Procédé d'analyse d'un signal du type représentable par une courbe portant des trains d'ondulations et présentant une dérive de la ligne de base, notamment pour l'analyse d'un signal de manométrie digestive, par exemple en manométrie colique,
procédé caractérisé en ce qu'il comprend une première étape de correction de la dérive de ladite ligne de base, consistant à identifier des minima significatifs (86) dans ladite courbe à trains d'ondulations parmi des points candidats, et à réaligner ladite courbe en relation avec lesdits minima significatifs identifiés (86), et une seconde étape d'analyse quantitative de ladite courbe ainsi réalignée,
et en ce que ladite étape d'identification des minima significatifs (86) consiste à identifier itérativement chaque nouveau minimum significatif $(86_{i+1})$ par explorations successives de points de ladite courbe suivant le dernier minimum significatif identifié $(86_i)$, un nouveau minimum significatif $(86_{i+1})$ étant identifié en prenant en compte, pour chaque point candidat courant exploré, au moins un des points candidats précédents $(85_j)$ et /ou suivants $(85_l)$, et en prenant ou non une décision de sélection, en tant que nouveau minimum significatif, dudit point courant exploré et/ou d'un desdits points candidats précédents ou suivants en comparant les pentes des droites reliant le dernier minimum significatif identifié $(86_i)$ à chacun desdits points candidats $(85_j ; 85_l)$ pris en compte et au point courant exploré $(86_{i+1})$ entre elles et avec la pente reliant les deux derniers minima significatifs identifiés $(86_{i-1}, 86_i)$.

2. Procédé selon la revendication 1 caractérisé en ce qu'un nouveau minimum significatif $(86_{i+1})$ est identifié lorsque tang s et tang mem sont de signes contraires, et tang s est négative ou nulle et/ou lorsque tang s et tang mem sont de signes contraires, et que tang p est strictement positive, tang mem étant défini comme la droite de liaison dudit dernier minimum significatif $(86_i)$ au point précédent exploré (85), tang s la droite de liaison dudit dernier minimum significatif $(86_i)$ au point courant exploré et tang p la droite de liaison des deux derniers minima significatifs.

3. Procédé selon la revendication 2 caractérisé en ce que ladite étape d'identification des minima significatifs consiste à identifier itérativement chaque nouveau minimum significatif par explorations successives de points de la courbe suivant le dernier minimum significatif $(86_i)$ identifié, un nouveau minimum significatif $(85_j, 86_{i+1})$ étant identifié en fonction de la valeur des pentes de droite de liaison $P_1$, $P_2$, $P_3$ et $P_4$, $P_1$ étant défini comme étant la pente de la droite de liaison entre les deux derniers minima significatifs identifiés $((86_{i-1}) ; 86_i))$ $P_2$ la pente de la droite de liaison du dernier minimum significatif $(86_i)$ au point précédemment exploré $(85_j)$, $P_3$ la pente de la droite de liaison du dernier minimum significatif $(86_i)$ au point courant exploré $(86_{i+1}+i)$ et $P_4$ la pente de la droite de liaison du dernier minimum significatif $(86_i)$ au point suivant exploré $(85_l)$.

4. Procédé selon la revendication 3 caractérisé en ce qu'un nouveau minimum significatif $X_{sign}(k+1)$ d'abcisse $X_{min}(i)$ ou $X_{min}(i-1)$ est identifié lorsque l'une des deux relations suivantes est respectivement vérifiée :
- **si** signe $P_2$ différent du signe $P_3$
si $P_1 \geqq 0$ et $P_3 > 0$
**alors** $X_{sign}(k+1) = X_{min}(i-1)$
**sinon**
si $P_1 \leqq 0$ et $P_3 < 0$ et $P_4 > P_3$
**alors** $X_{sign}(k+1) = X_{min}(i)$
- **sinon si** signe $P_2$ = signe $P_3$
si $(P_2 < 0$ et $P_3 < 0)$ et $(P_3 > P_2)$
**alors** $X_{sign}(k+1) = X_{min}(i-1)$
si $(P_2 > 0$ et $P_3 > 0)$ et $(P_3 < P_2)$ et $[(P_4 > P_3)$
**ou** durée tonus max $(X_{min}(i) - X_{sign}(k) > 1800)]$

alors $X_{sign}(k+1) = X_{min}(i)$
avec $X_{sign}(k) =$ dernier minimum significatif identifié.

5. Procédé selon l'une quelconque des revendications 1 à 4 caractérisé en ce que ladite étape de sélection desdits minima significatifs (86) est précédée d'une étape de sélection de points candidats sélectionnés en tant que mimina locaux (85) dans ladite courbe représentative du signal.

6. Procédé selon la revendication 5 caractérisé en ce que ladite sélection de minima candidats (85) consiste à découper ladite courbe en tronçons consécutifs, et à retenir, s'il existe, au moins un point d'ordonnée minimale dans une fenêtre autour d'une moyenne récursive pour chacun desdits tronçons.

7. Procédé selon la revendication 5 caractérisé en ce que ladite étape de sélection de minimas candidats consiste à détecter tous les minimas locaux par plage de 50 ou 100 points et à ne valider que ceux voisins d'une moyenne récursive calculée sur les minimas précédemment sélectionnés.

8. Procédé selon l'une quelconque des revendications 1 à 7 caractérisé en ce que ledit minimum significatif est choisi en fonction de la durée d'un tonus musculaire, notamment à une durée comprise entre environ 5 et environ 15 minutes, préférentiellement aux environs de 10 minutes.

9. Procédé selon la revendication 8 et l'une quelconque des revendications 5 et 6 caractérisé en ce que le nouveau minimum significatif ($86_{i+1}$) est choisi comme étant le premier minimum candidat sélectionné au-delà de la durée d'un tonus musculaire, lorsqu'aucun nouveau minimum significatif ($86_{i+1}$) n'a été identifié pendant ladite durée d'un tonus musculaire, à partir du dernier minimum significatif identifié ($86_i$).

10. Procédé selon l'une quelconque des revendications 2 à 4 et l'une quelconque des revendications 5 et 6 caractérisé en ce que l'initialisation de ladite étape d'identification desdits minima significatifs consiste à sélectionner comme première ligne de base, la droite réunissant les deux premiers minima candidats sélectionnés et/ou comme première valeur de la moyenne récursive, la valeur du minimum candidat sélectionné sur la première plage de points explorée.

11. Procédé selon la revendication 5 et l'une quelconque des revendications 6 à 10 caractérisé en ce que la durée d'un tronçon de sélection d'un minimum candidat, s'il existe, est comprise entre 15 secondes environ et 30 secondes environ.

12. Procédé selon l'une quelconque des revendications 1 à 11 caractérisé en ce que ladite étape de correction de la dérive de la ligne de base est précédée d'une étape de filtrage, comprenant une opération d'élimination des artefacts de ladite courbe et/ou une opération de filtrage à fenêtre.

13. Procédé selon la revendication 12 caractérisé en ce que ladite opération d'élimination desdits artefacts consiste à effectuer pour chaque point de la courbe, un calcul de la pente de la droite le liant aux points suivants de la courbe, et à initialiser le traitement d'élimination d'artefacts en cas de pente supérieure à un seuil prédéterminé,
ledit traitement d'élimination des artefacts consistant à éliminer tous les points de ladite courbe suivant le point d'initialisation et dont la droite les liant auxdits points d'initialisation présente une pente supérieure audit seuil prédéterminé.

14. Procédé selon l'une quelconque des revendications 1 à 13 caractérisé en ce que ladite étape de correction de la dérive de la ligne de base est suivie d'une étape de détection des zones d'activités, dans la courbe, comprenant une opération de détection des points de la courbe réalignée d'ordonnée inférieure à un seuil prédéterminé pour la détermination des zones de début et de fin d'activité.

15. Procédé selon la revendication 14 caractérisé en ce que ladite détection des zones d'activité comprend une opération de détermination de l'abcisse précise de début et de fin respectivement desdites zones de début et de fin d'activité, par seuillage des points environnants au moyen d'un second seuil prédéterminé, inférieur audit seuil de détermination des zones.

16. Procédé selon l'une quelconque des revendications 14 et 15 caractérisé en ce que ledit seuil de détermination des zones de début et de fin d'activité est d'environ 1/25 de la dynamique de la courbe, et/ou ledit second seuil est d'environ 1/3 de la valeur dudit seuil de détermination desdites zones.

17. Procédé selon l'une quelconque des revendications 14 à 16 caractérisé en ce que ladite étape de détection des zones d'activité est suivie par une étape d'approximation linéaire des zones d'activité détectées.

18. Procédé selon l'une quelconque des revendications 13 à 15 caractérisé en ce que lesdites valeurs de seuil prédéterminé sont ajustables.

Patrice VIDON Consultants
Univ. Rennes 1 / EERL
Dossier 1053A
DESSINS PROVISOIRES

repas

Fig. 1

*Patrice VIDON Consultants*
*Univ. Rennes 1 / EERL*
*Dossier 1053A*
*DESSINS PROVISOIRES*

Fig. 2

*Patrice VIDON Consultants*
*Univ. Rennes 1 / EERL*
*Dossier 1053A*
*DESSINS PROVISOIRES*

37  35

## Fig. 3A

38

39  36

## Fig. 3B

Signal original

## Fig. 4A

## Fig. 4B

Signal filtré

*Patrice VIDON Consultants*
*Univ. Rennes 1 / EERL*
*Dossier 1053A*
*DESSINS PROVISOIRES*

Fig. 5A

Fig. 5B

Signal aligné

Fig. 6A

Fig. 6B

Signal linéarisé

*Patrice VIDON Consultants*
*Univ. Rennes 1 / EERL*
*Dossier 1053A*
*DESSINS PROVISOIRES*

Fig. 7

*Patrice VIDON Consultants*
*Univ. Rennes 1 / EERL*
*Dossier 1053A*
*DESSINS PROVISOIRES*

Fig. 8A

Fig. 8B

Fig. 8C

Patrice VIDON Consultants
Univ. Rennes 1 / EERL
Dossier 1053A
DESSINS PROVISOIRES

Fig. 9A

85

86i

tangp

tangmem

tangs

minxx[i]

86i+1

Fig. 9B

85

86i

tangmem

tangp

tangs

86i+1

Fig. 9C

86i

86i+1

tangs

tangp

85

Index
Moteur

Activité

2000 — 100%

1600 — 80%

1200 — 60%

800 — 40%

90

91

400 — 20%

0

20  40  60  80  100  120  140  160  20  40  60  80  mn

92

93

Repas

Fig. 10

Patrice VIDON Consultants
Univ. Rennes 1 / EERL
Dossier 1053A
DESSINS PROVISOIRES

$86_{i-1}$  $P_1$  $86_i$  $85_j$  $85_i$  $P_2$  $P_3$  $86_{i+1}$  $P_4$  $85_\ell$

$X_{sign}(k-1)$  $X_{sign}(k)$  $X_{min}(i-1)$  $X_{min}(i+1)$

$X_{min}(i)$

Fig. 11

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | JOURNAL OF CHROMATOGRAPHIC SCIENCE, vol. 9, no. 12, 10 décembre 1971, pages 710-717; J.D. HETTINGER et al.: "A new computing integrator for chromatography" * Figure 4; pages 713-716, paragraphe: "Data interpretation"; figures 7-12 * | 1-18 | G 06 F 15/20 |
| D,A | AUTOMEDICA, vol. 7, no. 4, 1987, pages 221-236, Gordon and Breach Science Publishers Inc., GB; A.J. FLATT et al.: "Computer analysis of intestinal motor activity" * Pages 228-230, paragraphe: "Analysis of contractions" * | 1-18 | |
| D,A | GASTROENTEROL CLIN. BIOL., vol. 12, no. 4, 1988, pages 368-375, Masson, Paris, FR; C. ROZE: "Systèmes automatisés de traitement des signaux de motricité digestive" * Pages 369-370, paragraphe: "Problèmes posés par le traitement des signaux mécaniques"; page 372, paragraphe: "Signaux de manométrie ou mesurant les contractions pariétales de l'intestin grêle et du côlon" * | 1-18 | |
| A | MEDICAL & BIOLOGICAL ENGINEERING & COMPUTING, vol. 23, no. 2, mars 1985, pages 143-149, IFMBE, Londres, GB; M. SCHEMANN et al.: "Computerised method for pattern recognition of intestinal motility: functional significance of the spread of contractions" * Pages 144-146, paragraphe: "Pattern recognition method" *              -/- | 1-18 | |

DOMAINES TECHNIQUES RECHERCHES (Int. Cl.5)

G 06 F 15/20

Le présent rapport a été établi pour toutes les revendications

| Lieu de la recherche | Date d'achèvement de la recherche | Examinateur |
|---|---|---|
| LA HAYE | 23-05-1990 | LECOMTE J.M. |

EPO FORM 1503 03.82 (P0402)

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | IEEE TRANSACTIONS ON BIOMEDICAL ENGINEERING, vol. 29, no. 5, mai 1982, pages 309-314, IEEE, New York, US; R.W. SUMMERS et al.: "Computerized analysis of spike burst activity in the small intestine" <br> * En entier * | 1-18 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| LA HAYE | 23-05-1990 | LECOMTE J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
............
& : membre de la même famille, document correspondant

EPO FORM 1503 03.82 (P0402)